# FASCICULE DE BREVET EUROPEEN

(11) **EP 3 773 319 B1**
(45) Date de publication et mention de la délivrance du brevet: **19.06.2024**
(21) Numéro de dépôt: 19716890.9
(22) Date de dépôt: 09.04.2019
(51) Int. Cl.: A61C 7/00, A61C 7/18, A61C 7/10, A61F 5/50, A61C 7/22

(54) **DISJONCTEUR DENTAIRE**
DENTALEXPANDER
DENTAL EXPANDER

(30) Priorité: 09.04.2018 FR 1853065
(43) Date de publication de la demande: 17.02.2021
(73) Titulaire: D&D, 75011 Paris (FR)
(72) Inventeur: BENAROUCH, Dan, 45000 ORLEANS (FR); MAJBRUCH, Delphine, 75011 PARIS (FR)
(74) Mandataire: Cabinet Nony
(86) Numéro de dépôt international: PCT/EP2019/058945
(87) Numéro de publication internationale: WO 2019/197399

(56) Documents cités:
- WO-A1-2017/198640
- WO-A1-2019/200008
- US-A1- 2016 081 769
- US-A1- 2017 007 367
- US-A1- 2017 079 747

## Description

### Domaine technique

L'invention concerne un procédé de fabrication d'un disjoncteur dentaire.

### Art antérieur

Un dispositif dentaire est destiné à être fixé sur les dents d'un patient. Il comporte à cet effet un, deux ou plus de deux organes d'attache, chaque organe d'attache étant fixé rigidement, par exemple collé, à une dent, dite « dent de fixation ».

Il comporte également un organe actif connecté aux organes d'attache et qui a pour fonction d'exercer une action sur la ou les dents de fixation, généralement pour les déplacer l'une par rapport à l'autre. L'action peut être par exemple une traction.

Les organes d'attache sont typiquement choisis parmi les bagues de fixation dentaire et les attaches orthodontiques.

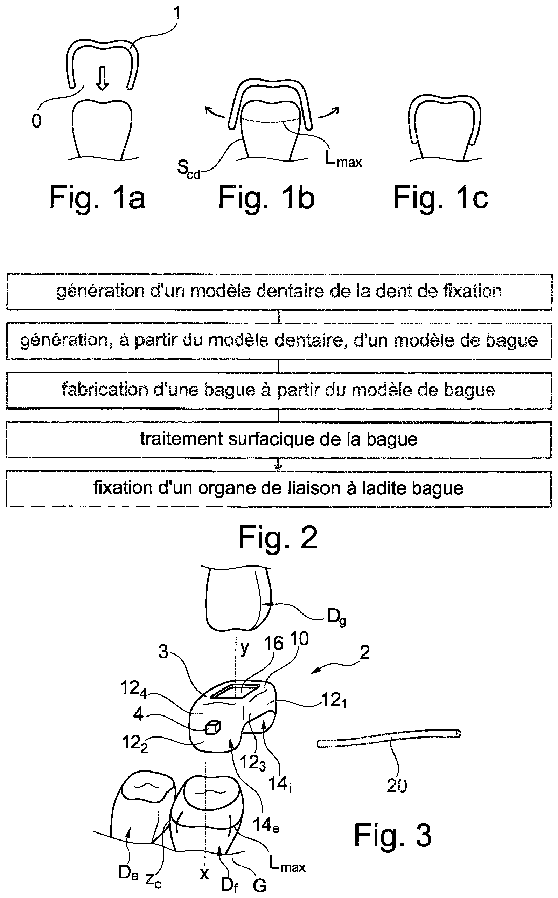

Une bague de fixation dentaire est une coque classiquement destinée à être fixée sur une molaire, par interposition d'une colle ou d'un ciment de scellement. A cet effet, la bague présente classiquement une déformabilité élastique qui lui permet d'être clipsée sur la dent de fixation. Plus précisément, comme illustré sur la figure 1a, la bague 1 définit une ouverture O dont les dimensions n'autorisent qu'un montage en force sur la dent de fixation. Comme illustré sur la figure 1b, lors du montage sur la dent de fixation, le bord définissant l'ouverture se déforme pour augmenter l'aire de ladite ouverture jusqu'au passage de la ligne de plus grand contour de la dent Lₘₐₓ. Après avoir passé cette ligne, ce bord revient élastiquement vers sa position d'origine, en prenant appui sur une surface en contre-dépouille S_{cd} de la dent de fixation, ce qui maintient en position la bague sur cette dent (figure 1c).

Une attache orthodontique, en anglais « *bracket* »*,* est destinée, comme la bague, à être fixée, classiquement collée, sur une dent. A la différence d'une bague, une attache orthodontique ne forme pas une coque qui entoure la dent de fixation.

Une attache orthodontique est généralement fixée sur une incisive, une canine ou une prémolaire, de préférence sur la face vestibulaire ou linguale des dents.

Une bague peut compléter un dispositif multi-attaches dans les secteurs postérieurs. En effet, les forces masticatoires exercées dans les secteurs postérieurs augmentent la probabilité de décollement. Les bagues se décollant moins facilement que les attaches orthodontiques, il est préférable de combiner bagues en postérieur et attaches orthodontiques sur les prémolaires, canines et incisives.

US2017007367A1 divulgue un appareil orthodontique fabriqué directement pour élargir un palais d'un patient, l'appareil comprenant : une partie d'engagement de dents comprenant une pluralité de structures d'engagement de dents ; une partie de génération de force couplée à la partie d'engagement de dents, la partie de génération de force comprend un polymère hydratable configuré pour se dilater au contact de la salive du patient.

Il existe un besoin permanent pour un dispositif dentaire mieux adapté aux besoins du patient et donc permettant un traitement plus efficace.

Il existe également un besoin pour un procédé de fabrication d'un dispositif dentaire limitant le nombre de rendez-vous chez l'orthodontiste, notamment pour concevoir et essayer les prototypes d'organes d'attache, et notamment de bagues.

Il existe enfin un besoin d'un procédé de fabrication pour un dispositif dentaire limitant les douleurs subies par le patient lors de la préparation de ce dispositif dentaire.

Un but de l'invention est de répondre, au moins partiellement, à ces besoins.

### Résumé de l'invention

On décrit un procédé de fabrication d'un dispositif dentaire comportant :
- un premier organe d'attache destiné à être fixé sur une première dent de fixation d'un patient ;
- un organe actif rigidement solidaire du premier organe d'attache ;
ledit procédé comportant les étapes successives suivantes :
a) génération d'un modèle numérique tridimensionnel d'une arcade dentaire comportant la dent de fixation, ou « modèle d'arcade », de préférence au moyen d'un scanner optique, d'un scanner à rayons X ou Cone beam CT, de préférence au moyen d'un scanner optique ;
b) génération, à partir du modèle d'arcade, et de préférence à partir d'un traitement à appliquer au patient :
   - d'un modèle numérique tridimensionnel du premier organe d'attache, le modèle du premier organe d'attache présentant une surface intérieure reproduisant sensiblement une partie de la surface de la première dent de fixation, et/ou
   - d'un modèle numérique tridimensionnel de l'organe actif ;
c) fabrication, de préférence par impression 3D,
   - à partir du modèle du premier organe d'attache, dudit premier organe d'attache, et/ou
   - à partir du modèle de l'organe actif, dudit organe actif ;
d) de préférence, traitement de l'organe d'attache de manière que sa surface intérieure présente une rugosité supérieure à la rugosité de sa surface extérieure.

Selon l'invention, le dispositif dentaire est un disjoncteur dentaire et le procédé est conforme à la revendication 1.

De préférence, à l'étape c), on simule par ordinateur l'action d'un dispositif dentaire fabriqué à partir du modèle du premier organe d'attache et/ou du modèle de l'organe actif, afin de définir
- la forme et/ou l'orientation d'une gorge du premier organe d'attache, et/ou
- la forme et/ou le matériau de constitution de l'organe actif, et/ou
- la forme d'un deuxième organe d'attache.

Le premier organe d'attache, imprimé par impression 3D, peut être une attache orthodontique ou une bague de fixation dentaire comportant un fond destiné à être posé sur une surface occlusale de la première dent de fixation, et une paroi latérale destinée à ceinturer ladite première dent de fixation, la paroi latérale étant constituée d'une paroi linguale, d'une paroi vestibulaire et de première et deuxième parois proximales.

Le premier organe d'attache est de préférence en un matériau choisi parmi les alliages de cobalt-chrome, le titane, les alliages de Nickel-Titane et les alliages de Titane-Niobium.

L'organe actif peut être venu de matière avec le premier organe d'attache ou y être attaché, généralement après fixation de l'organe d'attache.

Le dispositif dentaire comporte au moins des premier et deuxième organes d'attache et un organe actif qui s'étend entre les deux organes d'attache. L'organe actif peut alors être une traverse ou un organe de liaison selon qu'il est venu de matière avec au moins un organe d'attache, de manière à former un ensemble monobloc, ou non, respectivement. Autrement dit, l'organe de liaison est indépendant des organes d'attache avant d'y être fixé. Une traverse peut être également indépendante d'un organe d'attache avant d'y être fixée, mais elle forme un ensemble monobloc avec au moins un organe d'attache.

L'organe de liaison peut être rapporté pour être fixé sur les organes d'attaches, généralement après que ces organes d'attache ont été fixées sur les dents, respectivement. L'organe de liaison est alors initialement indépendant des organes d'attache.

L'organe de liaison peut être par exemple un arc orthodontique, par exemple à mémoire de forme, qui coulisse dans des gorges ou des fourreaux ménagés dans des organes d'attache.

De préférence, lorsqu'il sert à la fixation d'un organe de liaison, un organe d'attache porte une attache à cet effet. L'attache peut en particulier comporter une gorge dans laquelle l'organe de liaison est disposé avant d'être immobilisé.

L'organe de liaison peut être également soudé sur le premier organe d'attache, ce qui est particulièrement avantageux lorsque l'organe de liaison est réalisé sur mesures.

L'organe actif est de préférence choisi parmi :
- une traverse de liaison du premier organe d'attache avec un deuxième organe d'attache destiné à être fixé sur une deuxième dent de fixation du patient ;
- un organe de liaison du premier organe d'attache avec un deuxième organe d'attache, l'organe de liaison étant destiné à être rapporté et fixé sur le premier organe d'attache, notamment un arc orthodontique, par exemple un double arc de Delaire, un arc transpalatin ou un arc lingual.

De préférence, l'organe de liaison est en un matériau à mémoire de forme.

De préférence, l'organe de liaison est fabriqué sur mesures, de préférence par un prothésiste, ou est obtenu par pliage, de préférence par un orthodontiste, d'un organe préformé.

Lorsque l'organe actif est une traverse de liaison, le deuxième organe d'attache est de préférence venu de matière avec le premier organe d'attache et, de préférence, avec la traverse de liaison, c'est-à-dire forme avec eux un ensemble monobloc fabriqué simultanément, par impression 3D.

Un disjoncteur fabriqué selon un procédé selon l'invention peut être un dispositif dentaire multi-attaches monobloc fabriqué par impression 3D, comportant une pluralité d'organes d'attache et une traverse de liaison formant un ensemble monobloc avec les organes d'attache, dispositif dans lequel les organes d'attaches et la traverse de liaison sont définis en fonction de l'action à exercer spécifiquement sur les dents du patient.

La solidarisation de plusieurs dents entre elles peut avantageusement servir à assurer un ancrage dentaire et éviter tout mouvement dentaire non souhaité.

Par ailleurs, les dispositifs dentaires multi-attaches traditionnels destinés à la rétraction d'un groupe de dents ou aux tractions intermaxillaires ou à la désinclusion d'une dent utilisent, pour assurer un ancrage, des arcs orthodontiques en acier de grosse section, ce qui nécessite souvent un nivellement de l'arcade dentaire. Avec un dispositif dentaire multi-attaches monobloc selon l'invention, il est avantageusement possible de supprimer l'étape de nivellement. La durée du traitement orthodontique en est réduite. Le deuxième organe d'attache est fabriqué à l'étape c) par impression 3D à partir d'un modèle du deuxième organe d'attache généré à l'étape b) et présentant une surface intérieure reproduisant sensiblement une partie de la surface de la deuxième dent de fixation.

Le dispositif dentaire peut comporter un accessoire de préférence choisi parmi :
- une attache de premier organe de fixation, pour la fixation d'un organe de liaison ; et/ou
- un crochet d'attache d'un élastique de traction intermaxillaire ;
- un fourreau de passage d'un organe de liaison, le fourreau pouvant être vestibulaire ou lingual ;
- un mainteneur d'espace ;
- une butée vestibulaire.

L'accessoire est de préférence venu de matière avec le premier organe d'attache de manière à former un ensemble monobloc.

**Selon une première variante principale du procédé selon l'invention,** l'organe actif est en un matériau à mémoire de forme, de préférence choisi parmi les alliages de cobalt-chrome, le titane, les alliages de Nickel-Titane et les alliages de Titane-Niobium.

La forme de l'organe actif et les propriétés de mémoire de forme sont de préférence déterminées en fonction du traitement à appliquer au patient.

Les inventeurs ont découvert que le procédé selon la première variante principale de l'invention permet une action du dispositif dentaire parfaitement ciblée aux besoins du patient.

L'impression 3D permet également une fabrication rapide et autorise des configurations spécifiques.

De préférence, la mémoire de forme de l'organe actif est désactivée, classiquement par refroidissement, avant que l'organe actif ne soit fixé dans la bouche du patient. La température corporelle réactive alors la mémoire de forme, ce qui permet au dispositif dentaire d'appliquer l'action prévue, de préférence une action continue et personnalisée.

De préférence, les organes d'attache et l'organe actif forment un ensemble monobloc, de préférence fabriqué en un alliage à mémoire de forme. De préférence, un traitement thermique est cependant appliqué sur les organes d'attache afin de supprimer leur mémoire de forme.

**Selon une deuxième variante principale du procédé selon l'invention,** le procédé comporte, à l'étape c), le ménagement d'une gorge dans le premier organe d'attache afin que l'organe actif puisse y être fixé rigidement.

De préférence, l'orientation de la gorge est définie en fonction de l'action à exercer spécifiquement par l'organe actif sur les dents du patient.

De préférence, on simule par ordinateur une correction alvéolo-dentaire pour le patient, afin de définir la forme et/ou l'orientation de la gorge.

Après avoir ménagé ladite gorge, on loge ledit organe actif dans ladite gorge, puis on l'immobilise dans ladite gorge.

Les inventeurs ont découvert que le procédé selon la deuxième variante principale de l'invention permet une action du dispositif dentaire parfaitement ciblée aux besoins du patient. En outre, l'organe actif peut être avantageusement un organe de liaison conventionnel.

Un disjoncteur fabriqué selon un procédé selon l'invention peut être un dispositif dentaire multi-attaches fabriqué par impression 3D, comportant une pluralité d'organes d'attache et un organe actif, dispositif dans lequel les organes d'attache comportent des gorges de réception de l'organe actif définies en fonction de l'action à exercer spécifiquement par l'organe actif sur les dents du patient, un ou plusieurs des organes d'attache pouvant alternativement être fabriqués, par impression 3D, simultanément à l'organe actif.

Un tel dispositif dentaire multi-attaches présente les mêmes avantages qu'un dispositif multi-attaches monobloc décrit ci-dessus. En outre, l'utilisation d'un organe actif sous la forme d'un organe de liaison, amovible, offre la possibilité de retirer l'organe de liaison quand son action est terminée afin de le remplacer par un autre organe de liaison exerçant une correction différente, sans avoir à retirer les organes d'attache.

Enfin, lorsque le dispositif dentaire comporte plusieurs organes actifs, dont au moins un organe de liaison, par exemple pour assurer simultanément une action d'expansion palatine et une action d'alignement des dents, le changement de l'organe de liaison ne modifie pas substantiellement l'action du ou des autres organes actifs. Par exemple, l'action d'alignement d'un organe actif n'est avantageusement pas sensiblement affectée par le changement d'un organe de liaison agissant sur l'expansion palatine par un organe de liaison passif sur l'expansion palatine.

Selon l'invention, le premier organe d'attache et/ou le deuxième organe d'attache est une bague de fixation dentaire, et à l'étape b), on définit le modèle de bague de manière que la bague issue de l'étape c) ne présente pas de surface en contre-dépouille c'est-à-dire prenant appui, dans la position de service, sur une surface en contre-dépouille de la dent de fixation.

**Selon une troisième variante principale du procédé selon l'invention,** le premier organe d'attache est une bague et, à l'étape b), on définit le modèle de bague de manière que la bague issue de l'étape c) :
- ne s'étende pas, dans une position de service dans laquelle elle est fixée sur la première dent de fixation, sur une zone de contact de la première dent de fixation avec une dent adjacente ; et/ou
- ne s'étende pas, dans la position de service, au-delà de la ligne de plus grand contour de la première dent de fixation ou, de préférence, s'étende au-delà de la ligne de plus grand contour de la première dent de fixation sans définir de surface en contre-dépouille.

Les inventeurs ont découvert que le procédé selon la troisième variante principale de l'invention permet à la bague d'être positionnée sur la première dent de fixation sans avoir à repousser la ou les dents adjacentes à la dent de fixation, ni comprimer la surface de la première dent de fixation à proximité de la ligne de plus grand contour. Ainsi la bague respecte-t-elle mieux l'anatomie de la première dent de fixation.

L'absence de surface de la bague en contre-dépouille, contraire à la pratique habituelle, permet avantageusement une fixation sans clipsage de la bague, c'est-à-dire sans qu'il soit nécessaire que la bague vienne prendre appui sur les surfaces en contre-dépouille de la première dent de fixation.

Les inventeurs ont enfin constaté qu'après collage, la bague est avantageusement fixée de manière très fiable.

**Selon une quatrième variante principale du procédé selon l'invention,** le premier organe d'attache est une bague et, à l'étape b), le modèle de bague est défini de manière que la bague présente un fond traversé par au moins un trou.

Le fond de la bague est destiné à reposer sur la face occlusale de la première dent de fixation. Le ménagement d'un trou dans le fond de la bague limite avantageusement les interactions entre la bague et la dent antagoniste à la première dent de fixation.

Les inventeurs ont constaté que le risque de décollement de la bague en est considérablement réduit. En outre, la bague est mieux acceptée par le patient.

De préférence, le trou est défini de manière que, dans la position de service, le fond de la bague ne recouvre pas, même partiellement, la surface de mastication de la dent de fixation.

**Selon une cinquième variante principale du procédé selon l'invention,** le premier organe d'attache est une bague et le procédé comporte, après l'étape c), une étape d) de traitement surfacique de la bague. De préférence, à l'étape d), on polit au moins une partie de la surface extérieure de la bague, sans polir la surface intérieure de la bague.

Comme on le verra plus en détail dans la suite de la description, la rugosité de la surface intérieure de la bague, supérieure à celle de sa surface extérieure, favorise l'efficacité de la fixation de la bague sur la dent de fixation.

Bien entendu, les différentes caractéristiques, optionnelles ou non, des différentes variantes principales de l'invention, peuvent être combinées.

Selon l'invention, l'organe actif comporte, de préférence est constitué en un matériau à mémoire de forme ou en un matériau polymérique déformable sous l'effet d'un stimulus, de préférence sous l'effet d'une augmentation de température résultant de la mise du disjoncteur dans la position de service.

Quelle que soit la variante principale considérée, un procédé selon l'invention peut encore comporter une ou plusieurs des caractéristiques optionnelles suivantes :
- l'organe actif comporte, de préférence est constitué en un matériau à mémoire de forme ou en un matériau polymérique déformable sous l'effet d'un stimulus sous l'effet d'une augmentation de température résultant de la mise du disjoncteur dans la position de service ;
- après l'étape c), on désactive la mémoire de forme de l'organe actif par refroidissement avant que le disjoncteur ne soit fixé dans la position de service dans la bouche du patient, le matériau à mémoire de forme étant choisi de manière que la température corporelle du patient réactive ladite mémoire de forme ;
- à l'étape c), l'organe actif est de préférence venu de matière avec le premier et/ou deuxième organes d'attache de manière à former un ensemble monobloc ;
- à l'étape c), l'organe actif est fabriqué en un matériau identique à celui du premier organe d'attache et/ou du deuxième organe d'attache ;
- à l'étape d), on polit au moins une partie de la surface extérieure du premier organe d'attache, sans polir la surface intérieure du premier organe d'attache ;
- le premier organe d'attache est une bague et, à l'étape b), le modèle de bague est défini de manière que la hauteur de la paroi linguale et/ou de la paroi vestibulaire soit supérieure à la hauteur de la première paroi proximale et/ou de la deuxième paroi proximale ;
- le premier organe d'attache est une bague et, à l'étape b), le modèle de bague est défini de manière que la paroi linguale et/ou la paroi vestibulaire s'étendent, depuis le fond, au-delà de la ligne de plus grand contour de la dent de fixation ;
- le premier organe d'attache est une bague et, à l'étape b), lorsque la première paroi proximale est destinée à s'étendre, dans la position de service, entre la première dent de fixation et une dent adjacente en contact avec la première dent de fixation par une zone de contact, le modèle de bague est défini de manière que, dans ladite position de service, la première paroi proximale n'atteigne pas ladite zone de contact.

Dans un mode de réalisation, à l'étape c), on fabrique, par impression 3D, un dispositif dentaire comportant
- un ensemble d'organes d'attache,
- un organe actif reliant des organes d'attache dudit ensemble, et
- un support maintenant lesdits organes d'attache dans des positions relatives correspondant à leurs positions relatives dans une position de service dans laquelle lesdits organes d'attache sont fixés sur des dents de fixation respectives.

Le support est de préférence conformé pour autoriser un collage indirect du dispositif dentaire. Avantageusement, le positionnement dans la position de service et le collage en sont simplifiés.

Dans un mode de réalisation, le support est détachable après fixation des organes d'attache sur les dents.

Dans un autre mode de réalisation, il n'est pas détachable et reste donc présent lors du traitement du patient.

On décrit également un dispositif dentaire fabriqué suivant un procédé selon l'invention.

Le dispositif dentaire est un disjoncteur.

L'invention est particulièrement adaptée à un disjoncteur. En particulier, le vérin des disjoncteurs conventionnels, utilisés pour élargir latéralement le disjoncteur, est de préférence remplacé par un organe actif fabriqué selon l'invention, de préférence en un matériau à mémoire de forme. L'organe actif agit alors comme un ressort.

L'expansion du palais peut être ainsi personnalisée, la quantité d'expansion étant déterminée informatiquement à l'avance, de manière à fabriquer un organe actif adapté pour fournir cette quantité d'expansion.

Avantageusement, le disjoncteur est fixe et l'expansion se réalise sans action spécifique du patient.

### Définitions

La « surface de mastication » est la surface par laquelle la dent de fixation entre en contact avec une ou plusieurs dents de l'arcade antagoniste lors de la mastication, en l'absence de bague.

Une « fixation » est une solidarisation « définitive », c'est-à-dire qui n'autorise pas une désolidarisation, à volonté, par le patient, à la différence d'une gouttière orthodontique par exemple.

Par « modèle d'arcade », on entend un modèle représentant tout ou partie de ladite arcade.

On appelle classiquement « ligne de plus grand contour », la ligne qui définit le plus grand contour d'une dent, un contour étant une ligne s'étendant sur la surface extérieure de la dent, dans un plan transversal à l'axe X de la dent, et ceinturant la dent.

### Brève description des figures

D'autres caractéristiques et avantages de l'invention apparaîtront encore à la lecture de la description détaillée qui va suivre et à l'examen du dessin annexé dans lequel :
- la figure 1 illustre schématiquement les différentes étapes de la fixation d'une bague selon l'art antérieur ;
- la figure 2 représente schématiquement un procédé selon l'invention ;
- la figure 3 représente schématiquement la fixation d'une bague selon l'invention ;
- les figures 4a-4e représentent des exemples de dispositifs dentaires.

### Description détaillée

La figure 1 ayant été décrite en préambule, on se reporte aux figures 2 et 3.

Une bague 2 selon l'invention comporte une coque 3 pourvue d'une attache de bague 4 permettant la fixation d'un organe de liaison exerçant, dans la position de service, une force sur une dent de fixation D_{f} sur laquelle est collée la bague de fixation 2.

La bague 2 présente un fond 10, ou « paroi occlusale », destiné à prendre appui sur la surface occlusale de la dent de fixation, et une paroi latérale 12 s'étendant, dans la position de service, depuis le fond 10 vers la gencive G. Le fond 10 est percé d'un trou 16 adapté pour que, dans la position de service, la surface de mastication de la dent de fixation puisse entrer en contact avec une dent antagoniste D_{g}.

De préférence, le trou 16 est défini de manière que, dans la position de service, le fond de la bague ne recouvre pas, même partiellement, la surface de mastication de la dent de fixation. De préférence, l'aire du trou 16 est supérieure à 0,5 fois, de préférence supérieure à 0,8 fois, de préférence supérieure à 1,0 fois et/ou inférieure à 1,2, de préférence inférieure à 1,1 fois l'aire de la surface de mastication de la dent de fixation. La limitation de l'aire du trou permet avantageusement à la surface intérieure de la bague d'assurer une fixation efficace sur la dent de fixation.

La paroi latérale 12 est constituée d'une paroi linguale ou palatine 12₁ s'étendant, dans la position de service, en regard de la langue, d'une paroi vestibulaire 12₂ opposée à la paroi linguale 12₁ et faisant face, dans la position de service, aux joues et/ou aux lèvres, et de première et deuxième parois proximales 12₃ et 12₄, au moins une des parois proximales séparant, dans la position de service, la dent de fixation et une dent adjacente Dₐ.

On appelle « hauteur » d'une paroi la dimension de cette paroi, mesurée selon l'axe Y de la bague, depuis le fond 10 de la bague. De préférence, la hauteur de la paroi linguale 12₁ et/ou de la paroi vestibulaire 12₂ est supérieure à la hauteur de la première paroi proximale et/ou de la deuxième paroi proximale. De préférence, la hauteur de la paroi linguale 12₁ et/ou de la paroi vestibulaire 12₂ est supérieure à 1 mm et/ou inférieure à 15 mm. De préférence, la hauteur de la première paroi proximale et/ou de la deuxième paroi proximale est supérieure à 0.5 mm et/ou inférieure à 10 mm.

De préférence, les hauteurs des parois linguale et vestibulaire sont sensiblement identiques. Il en est de même pour les parois proximales.

Cependant, la paroi linguale 12₁ et/ou la paroi vestibulaire 12₂ s'étendent de préférence, depuis le fond 10, de manière à ne pas dépasser, dans la position de service, la ligne de plus grand contour Lₘₐₓ de la dent de fixation.

De préférence, lorsqu'une paroi proximale est destinée à s'étendre entre la dent de fixation D_{f} et une dent adjacente Dₐ en contact avec la dent de fixation par une zone de contact Z_{c}, la hauteur de cette paroi proximale est déterminée pour que, dans la position de service, elle n'atteigne pas la zone de contact Z_{c}. La fixation de la bague 2 ne nécessite donc pas de forcer un passage entre la dent de fixation D_{f} et la dent adjacente Dₐ.

Le fond 10 et la paroi latérale 12 forment ensemble une cuvette qui définit la surface intérieure 14i de la bague, orientée vers l'intérieur de la cuvette, et la surface extérieure 14ₑ, orientée vers l'extérieur de la cuvette.

De préférence, au moins une partie, de préférence toute la surface intérieure 14ᵢ est rugueuse au toucher, ce qui optimise l'efficacité du collage de la bague 2.

De préférence, au moins une partie, de préférence toute la surface extérieure 14ₑ est lisse au toucher, de préférence polie, ce qui améliore l'esthétique de la bague.

L'attache de bague 4 peut être quelconque. De préférence, elle présente la forme d'un étrier adapté pour, dans une position passive, recevoir un organe de liaison 20 et pour, dans une position active, serrer l'organe de liaison afin de le maintenir en position.

Classiquement, lors d'un traitement orthodontique, l'organe de liaison 20 est fixé sur deux bagues. Il est actif, et notamment configuré pour exercer une force tendant à rapprocher les deux bagues ou à les écarter l'une de l'autre.

L'organe de liaison peut être en particulier un arc orthodontique à mémoire de forme ou un ressort à mémoire de forme ou un vérin.

Une bague d'un disjoncteur dentaire fabriqué selon l'invention peut être en particulier fabriquée suivant les étapes décrites ci-dessus, notamment suivant le mode de réalisation préféré décrit ci-après.

Une bague selon l'invention peut être encore pourvue de :
- une traverse de liaison avec une autre bague ou avec une attache orthodontique ;
- un crochet pour attacher un élastique de traction intermaxillaire ;
- un fourreau de passage d'un arc orthodontique ;
- un mainteneur d'espace ;
- une butée vestibulaire, utile lors de la dépose de la bague.

Une bague d'un disjoncteur dentaire fabriqué selon l'invention est de préférence fabriquée ensemble avec le ou les accessoires, afin de constituer un dispositif dentaire monobloc.

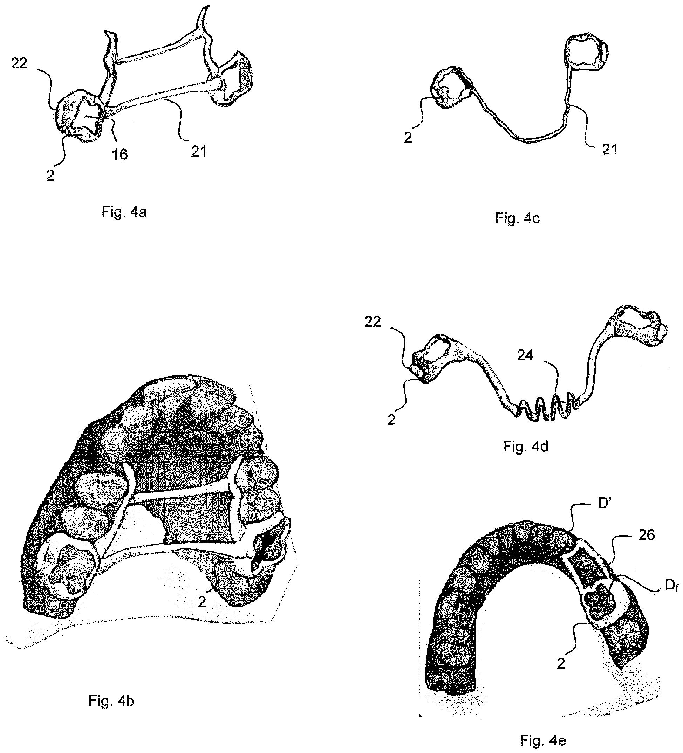

La figure 4a représente par exemple un disjoncteur dentaire selon l'invention comportant deux bagues 2 reliées par deux traverses de liaison 21 qui suivent la forme de la voûte dentaire. Les bagues portent des crochets 22. La figure 4b représente ce dispositif dans sa position de service.

La figure 4c représente un arc lingual, comportant deux bagues 2 reliées par une traverse de liaison 20 qui suit le contour de l'arcade. Ce dispositif peut être fixé à une arcade inférieure.

La figure 4d représente un dispositif dentaire comportant deux bagues 2 reliées par une traverse de liaison 21 qui porte une grille anti-pouce 24.

La figure 4e représente un dispositif dentaire hors invention, en l'occurrence un écarteur comportant une bague 2 fixée sur une dent de fixation D_{f} et pourvue d'un mainteneur d'espace 26 en appui sur une dent d'appui D' de la même arcade, de manière à maintenir écartées l'une de l'autre les dents de fixation et d'appui. Le mainteneur d'espace 26 a de préférence la forme d'un anneau.

Un mainteneur d'espace a pour fonction d'éviter toute version de dents adjacentes à une zone édentée, futur espace d'éruption d'une dent.

Tous ces dispositifs sont monoblocs et fabriqués chacun, en une unique opération, au moyen d'une imprimante 3D.

Dans un mode de réalisation préféré, une ou plusieurs attaches orthodontiques sont fabriquées par impression 3D, de préférence simultanément à la bague.

Une attache orthodontique peut être reliée à une bague par une traverse de liaison, l'ensemble constitué par la bague, la traverse de liaison et l'attache orthodontique formant un ensemble monobloc. Ce mode de réalisation est particulièrement utile pour améliorer l'ancrage dentaire dans le cadre de rétraction des dents du bloc incisivo-canin, ou pour utiliser des élastiques inter-maxillaires sans avoir à utiliser un appareil multi-attaches classique et surtout sans avoir besoin de niveler et d'aligner les dents au préalable. Le gain de temps qui en résulte est considérable.

Une attache orthodontique peut être reliée à une bague par un organe de liaison. Dans un appareil multi-attaches, unorgane de liaison est un arc orthodontique.

Le procédé est décrit ci-après dans le cadre de la fabrication d'une bague.

A **l'étape** a), l'orthodontiste scanne l'arcade dentaire comportant la dent de fixation D_{f}, de manière à générer un « modèle d'arcade ». Tous les scanners optiques conventionnels peuvent être utilisés.

De préférence, le modèle de l'arcade dentaire comportant la dent de fixation D_{f} est ensuite segmenté afin de générer un modèle numérique tridimensionnel de la dent de fixation, ou « modèle dentaire ». Dans un mode de réalisation, le modèle d'arcade sert de modèle dentaire.

De préférence, l'orthodontiste scanne également la dent antagoniste Dₐ, de préférence l'arcade antagoniste, ce qui lui permet de déterminer la position d'occlusion et ainsi de déterminer la surface de mastication de la dent de fixation. La surface de mastication peut être également déterminée par l'empreinte optique des arcades dentaires en occlusion. La surface de mastication peut également être personalisée par l'utilisation de systèmes de modélisation dynamique (exemple : société Modjaw).

A **l'étape** b), le modèle dentaire est analysé pour concevoir un modèle numérique tridimensionnel d'une bague selon l'invention, ou « modèle de bague », définissant une surface intérieure identique à la surface extérieure de la dent de fixation.

L'opérateur est de préférence une société spécialisée dans la conception et la fabrication des bagues. Elle travaille de préférence pour plusieurs orthodontistes, de préférence plus de 100, plus de 1000 ou plus de 10 000 orthodontistes.

De préférence, l'opérateur détermine la ligne de plus grand contour Lₘₐₓ de la dent de fixation. Le modèle d'arcade lui permet également de déterminer la ou les zones de contact Z_{c} éventuelles de la dent de fixation avec les dents adjacentes.

Connaissant la ou les zones de contact Z_{c}, la ligne de plus grand contour Lₘₐₓ et la surface de mastication de la dent de fixation, l'opérateur modélise alors la bague 2 afin que, dans sa position de service, sa surface intérieure 14i épouse étroitement la surface de la dent de fixation, sans s'étendre au-delà de la ligne de plus grand contour Lₘₐₓ, ni atteindre la ou les zones de contact Z_{c}. De préférence, l'opérateur crée un modèle de bague comportant un trou dont les dimensions et la position sont définies pour que la bague ne recouvre pas la surface de mastication de la dent de fixation. Toutes les techniques classiques de modélisation peuvent être utilisées à cet effet.

Cette forme du modèle de bague empêche le clipsage de la bague fabriquée à l'étape c) sur la dent de fixation, tout en facilitant sa mise en position de service, puis son acceptation par le patient.

De préférence, le modèle de bague est déterminé de manière qu'au moins une des parois linguale et vestibulaire de la bague, de préférence chacune de ces parois, s'étende, dans la position de service, vers la gencive, sensiblement jusqu'à la ligne de plus grand contour, de préférence en la dépassant, de préférence jusqu'à la jonction gingivo-dentaire, mais sans définir de surface en contre-dépouille. De préférence, ces parois s'étendent au-delà de la zone de contact avec la ou les dents adjacentes. La surface de collage en est avantageusement accrue.

De préférence, la conception du modèle de bague est assistée par ordinateur, notamment pour déterminer des épaisseurs de matériau adaptées aux contraintes mécaniques dans la position de service de la bague.

**A l'étape** c), l'opérateur fabrique la bague 2, au moyen d'une imprimante 3D, afin qu'elle soit conforme au modèle de bague créé à l'étape b).

De préférence, l'épaisseur et la nature du matériau constitutif de la coque sont définies de manière que la bague ne soit pas déformable élastiquement, et en particulier ne soit pas déformable à la main. Elle peut être notamment en un matériau choisi parmi les alliages Cobalt-Chrome, le titane, le nickel-titane, le titane-niobium ou tous les alliages métalliques biocompatibles. La bague de fixation n'a pas pour objet de remplacer tout ou partie de la dent de fixation, même de manière provisoire. Son épaisseur peut être sensiblement constante.

Dans un mode de réalisation préféré, l'épaisseur du fond de la bague est variable, de préférence pour diminuer progressivement en direction de son centre, de préférence de manière de manière à être sensiblement nulle au bord du trou 16.

L'utilisation de l'impression 3D pour fabriquer la bague permet avantageusement d'obtenir une rugosité de la surface intérieure adaptée à un collage efficace, sans autre opération de traitement.

La fabrication au moyen d'une imprimante 3D est avantageusement très rapide. Dans un mode de réalisation, la bague est réalisée immédiatement après l'étape a) et l'étape b), ce qui permet d'accélérer le traitement orthodontique.

Dans un mode de réalisation, préféré, la traverse de liaison est fabriquée, au moyen d'une imprimante 3D, en même temps que la bague. De préférence, la traverse de liaison et plusieurs bagues sont fabriqués, au moyen d'une imprimante 3D, en même temps. La pose sur l'arcade dentaire en est avantageusement facilitée.

De préférence encore, le ou les accessoires sont également fabriqués, au moyen d'une imprimante 3D, en même temps que la bague.

Dans un mode de réalisation, une gorge est usinée dans la bague afin qu'un organe de liaison puisse y être logé. De préférence, la gorge est définie en fonction du traitement à appliquer au patient. En particulier, l'orientation de la gorge est de préférence définie en fonction de l'action à exercer spécifiquement sur les dents du patient.

La fabrication combine ainsi une impression additive pour la bague, puis une fabrication soustractive, de préférence par usinage, pour la gorge.

De préférence, l'orientation de la gorge contient des informations de 3^{e} ordre qui s'exprimeront au moyen d'un arc orthodontique de section sensiblement complémentaire à celle de la gorge, de préférence de section carrée ou rectangulaire. Plus la section de l'arc orthodontique se rapproche de la section de la gorge, plus les informations de 3^{e} ordre se préciseront.

**A l'étape d),** l'opérateur polit de préférence la surface extérieure 14ₑ de la bague. Avantageusement, la surface extérieure 14ₑ présente ainsi une rugosité inférieure à la surface intérieure 14i, plus proche de la rugosité d'une dent.

Dans un mode de réalisation préféré, la surface intérieure n'est pas polie. La microstructure rugueuse de la surface intérieure 14i, résultant de l'impression 3D, est donc préservée.

Dans un mode de réalisation, pour augmenter la rugosité, on applique un rayon laser sur la surface intérieure afin de créer des micro-reliefs, et ainsi d'augmenter la rugosité.

Pour fixer la bague, l'orthodontiste dépose classiquement de la colle ou un ciment de scellement sur la surface intérieure 14i de la bague et/ou sur la dent de fixation. Toutes les colles ou ciments de scellement utilisés classiquement en orthodontie peuvent être utilisés.

Lorsque la bague s'étend au-delà de la ligne de plus grand contour de la dent de fixation sans définir intérieurement de surface en contre-dépouille, sa surface intérieure s'écarte de la surface en contre-dépouille de la dent de fixation, au-delà de cette ligne (en direction gingivale). De préférence, on ajuste la quantité de colle ou de ciment de scellement de manière à venir combler la région en contre-dépouille qui s'étend, au-delà de la ligne de plus grand contour, entre la surface intérieure de la bague et la surface de la dent de fixation.

L'orthodontiste vient ensuite appliquer la bague sur la dent de fixation. La forme de la surface intérieure 14ᵢ, identique à celle de la dent de fixation, permet une fixation précise et rapide, sans exercer de force importante sur la dent de fixation. La rugosité de la surface intérieure 14i améliore l'adhésion.

Après avoir posé une deuxième bague, non représentée, sur une deuxième dent de fixation, l'orthodontiste positionne l'organe de liaison 20 dans les attaches des deux bagues, puis active ces attaches de bague afin d'assurer une liaison rigide entre l'organe de liaison 20 et chacune des deux bagues.

En fonction de la situation clinique, la deuxième bague peut être remplacée par une attache orthodontique.

L'organe de liaison 20 peut ensuite assurer un effort, en traction ou en compression, sur les dents de fixation des bagues afin d'assurer des déplacements conformes au traitement orthodontique souhaité.

Les inventeurs ont constaté qu'une bague selon l'invention permet d'assurer un ancrage très efficace sur la dent de fixation, même si la bague n'est pas compressée par une dent adjacente et ne présente pas de paroi venant prendre appui sur une surface en contre-dépouille de la dent de fixation.

Le procédé décrit en détail ci-dessus peut également être mis en oeuvre pour fabriquer l'organe actif, et en particulier l'organe de liaison ou une traverse.

De préférence, le modèle numérique tridimensionnel de l'organe actif est conçu en fonction du traitement à appliquer. En particulier, il est de préférence constitué en un matériau à mémoire de forme et conformé en fonction des forces à exercer sur les dents du patient, ces forces étant dépendantes de la configuration spécifique des dents du patient. L'organe actif se substitue à un vérin d'un disjoncteur conventionnel et peut être en un matériau à mémoire de forme, mais également en un matériau polymère et/ou polymérique dit « 4D », c'est-à-dire programmable pour se déformer par application d'une augmentation de la température résultant de la mise en position de service, c'est-à-dire d'une augmentation de la température entre 20°C (température ambiante) ou une température inférieure à 20°C et environ 37°C.

Dans le cas d'un disjoncteur, le matériau de l'organe actif, de préférence le matériau du disjoncteur, est de préférence choisi pour, après activation du fait d'un stimulus, de préférence du fait de l'augmentation de température, tendre à élargir l'arcade dentaire, ce qui est la fonction classique d'un disjoncteur.

De préférence, l'organe actif comporte, voire est constitué en un matériau rétractable ou expansible sous l'effet de ladite augmentation de température, de préférence un matériau polymère. De préférence, la modification du volume de l'organe actif sous l'effet de cette augmentation de température est supérieure à 1%, à 2%, à 5%, à 8%, ou à 10%, à 20%, voire à 50% du volume initial, avant ladite augmentation de température.

L'organe peut être configuré pour se déformer progressivement ou instantanément sous l'effet ladite augmentation de température.

De manière générale, le stimulus d'activation de l'organe actif est choisi dans le groupe constitué par un rayonnement, en particulier lumineux, infrarouge, ultraviolet ou sonore, une modification de l'humidité et/ou de l'acidité et/ou de la température et/ou de la composition chimique de l'environnement de l'organe.

Dans un mode de réalisation, le stimulus est appliqué pendant une durée inférieure à 1 heure, à 30 minutes, à 60 s, à 30 s, à 10 s, à 5 s, à 1 s.

La pose d'un dispositif dentaire selon l'invention ne pose pas de difficulté particulière.

Dans un mode de réalisation, le dispositif dentaire est fabriqué, par impression 3D, avec un support qui maintient tous les organes d'attache dans des positions relatives correspondant à leurs positions relatives en service. Les organes d'attache peuvent être ainsi fixés sur les dents, de préférence collés, simultanément et avec une grande précision. Dans un mode de réalisation, le support est imprimé dans le même matériau que les organes d'attache. De préférence, il forme avec eux un ensemble monobloc. Après fixation sur les dents, le support peut être désolidarisé des organes d'attache en meulant les jonctions entre le support et les organes d'attache.

Dans un mode de réalisation, le support, de préférence au moins une dite jonction, est flexible, ce qui facilite la pose du dispositif dentaire, en particulier si un organe d'attache définit un intrados destiné à être en contact avec une surface dentaire en contre-dépouille. La flexibilité peut être ajustée en modifiant la section des jonctions.

Comme cela apparaît clairement à présent, l'invention permet un traitement orthodontique rapide, minimisant le nombre de rendez-vous chez l'orthodontiste, en particulier pour poser des séparateurs puis essayer des bagues.

Elle permet également de fabriquer un dispositif dentaire sur mesure.

La personnalisation du dispositif dentaire permet également de réduire les temps de finition et donc de raccourcir la durée globale d'un traitement.

Enfin, la réalisation et la pose des organes d'attache est indolore.

Bien entendu, l'invention n'est pas limitée aux modes de réalisation décrits et représentés, fournis à des fins illustratives uniquement.

En particulier, un organe d'attache, et notamment une bague, peut s'étendre sur plusieurs dents de fixation, notamment pour définir un point sensiblement immobile pour attacher l'organe de liaison.

Le dispositif dentaire peut comporter plusieurs organes actifs (traverses et/ou organes de liaison) fixés dans des fourreaux ou des gorges respectifs, un fourreau ou une gorge pouvant être vestibulaire ou lingual. Dans un mode de réalisation, le dispositif comporte un premier organe actif, fixé sur des premiers organes d'attache, au niveau du palais, pour créer de l'expansion palatine ou pour la maintenir, et un deuxième organe actif, sous la forme d'un arc orthodontique, fixé sur des deuxièmes organes d'attache, de préférence des gorges, pour modifier la position des dents. Il est donc possible de combiner deux actions de traitement : expansion palatine et alignement des dents.

## Revendications

1. Procédé de fabrication d'un disjoncteur dentaire comportant :
- un premier organe d'attache (2) destiné à être fixé sur une première dent de fixation d'une arcade dentaire d'un patient ;
- un deuxième organe d'attache destiné à être fixé sur une deuxième dent de fixation de l'arcade dentaire du patient ;
- un organe actif (21) rigidement solidaire des premier et deuxième organes d'attache et configuré pour, dans une position de service dans laquelle le disjoncteur est fixé sur ladite arcade dentaire, exercer une action d'élargissement de ladite arcade dentaire ;
le premier organe d'attache et/ou le deuxième organe d'attache étant une bague de fixation dentaire comportant un fond (10) destiné à être posé sur une surface occlusale d'une dent de fixation (D_{f}), et une paroi latérale (12) destinée à ceinturer ladite dent de fixation, la paroi latérale étant constituée d'une paroi linguale (12₁), d'une paroi vestibulaire (12₂) et de première et deuxième parois proximales (12₃,12₄), ledit procédé comportant les étapes successives suivantes :
a) génération, par ordinateur, d'un modèle numérique tridimensionnel d'une arcade dentaire comportant la dent de fixation, ou « modèle d'arcade », de préférence au moyen d'un scanner optique ;
b) génération par ordinateur, à partir du modèle d'arcade et à partir d'un traitement à appliquer au patient :
- d'un modèle numérique tridimensionnel du premier organe d'attache (2), le modèle du premier organe d'attache présentant une surface intérieure reproduisant sensiblement une partie de la surface de la première dent de fixation, et/ou
- d'un modèle numérique tridimensionnel du deuxième organe d'attache, le modèle du deuxième organe d'attache présentant une surface intérieure reproduisant sensiblement une partie de la surface de la deuxième dent de fixation, et/ou
- d'un modèle numérique tridimensionnel de l'organe actif (21) ;
c) fabrication au moyen d'une imprimante 3D,
- à partir du modèle du premier organe d'attache, dudit premier organe d'attache, et
- à partir du modèle du deuxième organe d'attache, dudit deuxième organe d'attache, et
- à partir du modèle de l'organe actif, dudit organe actif,
procédé dans lequel, à l'étape b), on définit le modèle de bague de manière que la bague issue de l'étape c) ne présente pas de surface en contre-dépouille ; et
l'organe actif (21) comporte
- un matériau à mémoire de forme ou
- un matériau polymérique déformable sous l'effet d'un stimulus d'activation, le stimulus d'activation de l'organe actif (21) étant choisi dans le groupe constitué par un rayonnement, une modification de l'humidité, une modification de l'acidité, une modification de la température, et une modification de la composition chimique de l'environnement de l'organe.

2. Procédé selon la revendication immédiatement précédente, dans lequel le stimulus d'activation de l'organe actif (21) est une augmentation de température résultant de la mise du disjoncteur dans la position de service.

3. Procédé selon l'une quelconque des deux revendications immédiatement précédentes, dans lequel, après l'étape c), on désactive la mémoire de forme de l'organe actif par refroidissement avant que le disjoncteur ne soit fixé dans la bouche du patient, le matériau à mémoire de forme étant choisi de manière que la température corporelle du patient active ladite mémoire de forme.

4. Procédé selon l'une quelconque des revendications précédentes, dans lequel, à l'étape c), l'organe actif (21) est venu de matière avec le premier et/ou deuxième organes d'attache (2) de manière à former un ensemble monobloc.

5. Procédé selon l'une quelconque des revendications précédentes, dans lequel à l'étape c), l'organe actif est fabriqué en un matériau identique à celui du premier organe d'attache et/ou du deuxième organe d'attache.

6. Procédé selon l'une quelconque des revendications précédentes, dans lequel, à l'étape b), on définit le modèle de bague de manière que la bague issue de l'étape c) s'étende, en direction gingivale, au-delà de la ligne de plus grand contour de la dent de fixation sans définir de région en contre-dépouille.

7. Procédé selon la revendication immédiatement précédente, dans lequel le modèle de bague est défini de manière que le fond (10) de la bague (2) soit traversé par au moins un trou (16).

8. Procédé selon la revendication immédiatement précédente, dans lequel le trou (16) est défini de manière que, dans la position de service, le fond de la bague ne recouvre pas, même partiellement, la surface de mastication de la première dent de fixation.

9. Procédé selon l'une quelconque des revendications précédentes, comportant, après l'étape c), une étape d) dans laquelle on polit au moins une partie de la surface extérieure de l'organe d'attache, sans polir la surface intérieure de l'organe d'attache.

10. Procédé selon l'une quelconque des revendications immédiatement précédentes, dans lequel, à l'étape b), le modèle de bague est défini de manière que la hauteur de la paroi linguale (12₁) et/ou de la paroi vestibulaire (12₂) soit supérieure à la hauteur de la première paroi proximale et/ou de la deuxième paroi proximale.

11. Procédé selon la revendication immédiatement précédente, dans lequel, à l'étape b), le modèle de bague est défini de manière que la paroi linguale (12₁) et/ou la paroi vestibulaire (12₂) s'étendent, en direction gingivale, depuis le fond (10), au-delà de la ligne de plus grand contour (Lₘₐₓ) de la dent de fixation.

12. Procédé selon l'une quelconque des revendications immédiatement précédentes, dans lequel, à l'étape b), lorsque la première paroi proximale est destinée à s'étendre, dans la position de service, entre la première dent de fixation (D_{f}) et une dent adjacente (Dₐ) en contact avec la première dent de fixation par une zone de contact (Z_{c}), le modèle de bague est défini de manière que, dans ladite position de service, la première paroi proximale n'atteigne pas ladite zone de contact.

13. Procédé selon l'une quelconque des revendications précédentes, dans lequel l'organe actif (21) est constitué en ledit matériau à mémoire de forme ou en ledit matériau polymérique déformable sous l'effet d'un stimulus d'activation.

14. Procédé selon l'une quelconque des revendications précédentes, dans lequel, après l'étape c), on dépose une colle ou un ciment de scellement sur la surface intérieure (14i) de la bague.

15. Procédé selon la revendication 14, la revendication 6 s'appliquant, dans lequel on ajuste la quantité de colle ou de ciment de scellement de manière à venir combler la région qui s'étend, au-delà de la ligne de plus grand contour, entre la surface intérieure de la bague et la surface de la dent de fixation.

## Patentansprüche

1. Verfahren zur Herstellung eines Dentalexpanders, welcher umfasst:
- ein erstes Befestigungselement (2), das dazu bestimmt ist, an einem ersten Befestigungszahn eines Zahnbogens eines Patienten befestigt zu werden;
- ein zweites Befestigungselement, das dazu bestimmt ist, an einem zweiten Befestigungszahn des Zahnbogens des Patienten befestigt zu werden;
- ein aktives Element (21), das starr mit dem ersten und zweiten Befestigungselement verbunden ist und dafür ausgelegt ist, in einer Gebrauchsposition, in welcher der Zahnexpander am Zahnbogen befestigt ist, eine Spreizwirkung auf den Zahnbogen auszuüben;
wobei das erste Befestigungselement und/oder das zweite Befestigungselement eine Zahnbefestigungshülse ist, die einen Boden (10), der dazu bestimmt ist, auf eine okklusale Fläche eines Befestigungszahns (D_{f}) aufgesetzt zu werden, und eine Seitenwand (12), die dazu bestimmt ist, den Befestigungszahn zu umschließen, umfasst, wobei die Seitenwand aus einer lingualen Wand (12₁), einer vestibulären Wand (12₂) und einer ersten und einer zweiten proximalen Wand (12₃,12₄) besteht, wobei das Verfahren die folgenden aufeinander folgenden Schritte umfasst:
a) Erzeugung, durch einen Computer, eines dreidimensionalen numerischen Modells eines den Befestigungszahn umfassenden Zahnbogens, oder eines "Zahnbogenmodells", vorzugsweise mittels eines optischen Scanners;
b) Erzeugung, durch einen Computer, anhand des Zahnbogenmodells und ausgehend von einer bei dem Patienten anzuwendenden Behandlung:
- eines dreidimensionalen numerischen Modells des ersten Befestigungselements (2), wobei das Modell des ersten Befestigungselements eine Innenfläche aufweist, die im Wesentlichen einen Teil der Oberfläche des ersten Befestigungszahns reproduziert, und/oder
- eines dreidimensionalen numerischen Modells des zweiten Befestigungselements, wobei das Modell des zweiten Befestigungselements eine Innenfläche aufweist, die im Wesentlichen einen Teil der Oberfläche des zweiten Befestigungszahns reproduziert, und/oder
- eines dreidimensionalen numerischen Modells des aktiven Elements (21);
c) Herstellung, mittels eines 3D-Druckers,
- anhand des Modells des ersten Befestigungselements, des ersten Befestigungselements, und
- anhand des Modells des zweiten Befestigungselements, des zweiten Befestigungselements, und
- anhand des Modells des aktiven Elements, des aktiven Elements,
wobei bei diesem Verfahren in Schritt b) das Modell der Hülse so definiert wird, dass die in Schritt c) erhaltene Hülse keine Hinterschneidungsfläche aufweist; und das aktive Element (21) umfasst:
- ein Formgedächtnismaterial oder
- ein Polymermaterial, das unter der Einwirkung eines aktivierenden Stimulus verformbar ist, wobei der aktivierende Stimulus des aktiven Elements (21) aus der Gruppe ausgewählt ist, die aus einer Bestrahlung, einer Änderung der Feuchtigkeit, einer Änderung des Säuregehalts, einer Änderung der Temperatur und einer Änderung der chemischen Zusammensetzung der Umgebung des Elements besteht.

2. Verfahren nach dem unmittelbar vorhergehenden Anspruch, wobei der aktivierende Stimulus des aktiven Elements (21) eine Temperaturerhöhung ist, die daraus resultiert, dass der Expander in die Gebrauchsposition gebracht wird.

3. Verfahren nach einem der zwei unmittelbar vorhergehenden Ansprüche, wobei nach Schritt c) das Formgedächtnis des aktiven Elements durch Abkühlung deaktiviert wird, bevor der Expander im Mund des Patienten befestigt wird, wobei das Formgedächtnismaterial so gewählt wird, dass die Körpertemperatur des Patienten das Formgedächtnis aktiviert.

4. Verfahren nach einem der vorhergehenden Ansprüche, wobei in Schritt c) das aktive Element (21) stoffschlüssig mit dem ersten und/oder zweiten Befestigungselement (2) verbunden ist, so dass eine einstückige Einheit gebildet wird.

5. Verfahren nach einem der vorhergehenden Ansprüche, wobei in Schritt c) das aktive Element aus einem Material hergestellt wird, das mit demjenigen des ersten Befestigungselements und/oder des zweiten Befestigungselements identisch ist.

6. Verfahren nach einem der vorhergehenden Ansprüche, wobei in Schritt b) das Modell der Hülse so definiert wird, dass sich die in Schritt c) erhaltene Hülse in gingivaler Richtung über die Linie der größten Kontur des Befestigungszahns hinaus erstreckt, ohne einen Hinterschneidungsbereich zu definieren.

7. Verfahren nach dem unmittelbar vorhergehenden Anspruch, wobei das Modell der Hülse so definiert wird, dass der Boden (10) der Hülse (2) von mindestens einem Loch (16) durchquert wird.

8. Verfahren nach dem unmittelbar vorhergehenden Anspruch, wobei das Loch (16) so definiert ist, dass in der Gebrauchsposition der Boden der Hülse die Kaufläche des ersten Befestigungszahns nicht und auch nicht teilweise bedeckt.

9. Verfahren nach einem der vorhergehenden Ansprüche, welches nach Schritt c) einen Schritt d) umfasst, in dem wenigstens ein Teil der Außenfläche des Befestigungselements poliert wird, ohne die Innenfläche des Befestigungselements zu polieren.

10. Verfahren nach einem der unmittelbar vorhergehenden Ansprüche, wobei in Schritt b) das Modell der Hülse so definiert wird, dass die Höhe der lingualen Wand (12₁) und/oder der vestibulären Wand (12₂) größer als die Höhe der ersten proximalen Wand und/oder der zweiten proximalen Wand ist.

11. Verfahren nach dem unmittelbar vorhergehenden Anspruch, wobei in Schritt b) das Modell der Hülse so definiert wird, dass die linguale Wand (12₁) und/oder die vestibuläre Wand (12₂) sich in gingivaler Richtung vom Boden (10) über die Linie der größten Kontur (Lₘₐₓ) des Befestigungszahns hinaus erstrecken.

12. Verfahren nach einem der unmittelbar vorhergehenden Ansprüche, wobei in Schritt b), wenn die erste proximale Wand dazu bestimmt ist, sich in der Gebrauchsposition zwischen dem ersten Befestigungszahn (D_{f}) und einem benachbarten Zahn (Dₐ) zu erstrecken, der sich mit dem ersten Befestigungszahn durch einen Kontaktbereich (Z_{c}) in Kontakt befindet, das Modell der Hülse so definiert wird, dass in der Gebrauchsposition die erste proximale Wand den Kontaktbereich nicht erreicht.

13. Verfahren nach einem der vorhergehenden Ansprüche, wobei das aktive Element (21) aus dem Formgedächtnismaterial oder aus dem Polymermaterial, das unter der Einwirkung eines aktivierenden Stimulus verformbar ist, besteht.

14. Verfahren nach einem der vorhergehenden Ansprüche, wobei nach Schritt c) ein Klebstoff oder ein Befestigungszement auf die Innenfläche (14ᵢ) der Hülse aufgebracht wird.

15. Verfahren nach Anspruch 14 in Verbindung mit Anspruch 6, wobei die Menge an Klebstoff oder Befestigungszement so angepasst wird, dass sie dann den Bereich ausfüllt, der sich über die Linie der größten Kontur hinaus zwischen der Innenfläche der Hülse und der Oberfläche des Befestigungszahns erstreckt.

## Claims

1. A method for manufacturing a dental expander having:
- a first bracket member (2) intended to be fixed to a first fixation tooth of a dental arch of a patient;
- a second bracket member intended to be fixed to a second fixation tooth of the dental arch of the patient;
- an active member (21) rigidly connected to the first and second bracket members and configured such that, in a service position in which the expander is fixed to said dental arch, it exerts an action that widens said dental arch;
the first bracket member and/or the second bracket member being a dental fixation ring having a bottom (10) intended to be placed on an occlusal surface of the first fixation tooth (D_{f}), and a lateral wall (12) intended to encircle said first fixation tooth, the lateral wall being composed of a lingual wall (12₁), a vestibular wall (12₂) and first and second proximal walls (12₃, 12₄),
said method having the following successive steps:
a) a three-dimensional digital model of a dental arch having the fixation tooth, or "arch model", is generated, preferably by means of an optical scanner;
b) the arch model and a treatment to be applied to the patient are used as a basis for generating:
- a three-dimensional digital model of the first bracket member (2), the model of the first bracket member having an inner surface that substantially reproduces a part of the surface of the first fixation tooth, and/or
- a three-dimensional digital model of the second bracket member, the model of the second bracket member having an inner surface that substantially reproduces a part of the surface of the second fixation tooth, and/or
- a three-dimensional digital model of the active member (21);
c) the model of the first bracket member is used as a basis for manufacturing said first bracket member, and/or the model of the second bracket member is used as a basis for manufacturing said second bracket member, and/or the model of the active member is used as a basis for manufacturing said active member, in each case by 3D printing,
in which method, in step b), the ring model is defined in such a way that the ring resulting from step c) does not have an undercut region; and
in which the active member (21) has
- a shape-memory material or
- a polymer material deformable under the effect of an activation stimulus,
the stimulus for activating the active member (21) being chosen from the group consisting of radiation, a change of the humidity, a change of the acidity, a change of the temperature, and a change of the chemical composition of the environment of the member.

2. The method as claimed in the immediately preceding claim, in which the stimulus for activating the active member (21) is a temperature increase resulting from the expander being placed in the service position.

3. The method as claimed in any one of the two immediately preceding claims, in which, after step c), the shape memory of the active member is deactivated by cooling before the expander is fixed in the mouth of the patient, the shape-memory material being chosen such that the body temperature of the patient activates said shape memory.

4. The method as claimed in any one of the preceding claims, in which, in step c), the active member (21) is made integral with the first and/or second bracket member so as to form a one-piece assembly.

5. The method as claimed in any one of the preceding claims, in which, in step c), the active member is manufactured from a material identical to that of the first bracket member and/or of the second bracket member.

6. The method as claimed in any one of the preceding claims,
in which method, in step b), the ring model is defined in such a way that the ring resulting from step c) extends beyond the line of greatest contour of the fixation tooth without defining an undercut region.

7. The method as claimed in the immediately preceding claim, in which the ring model is defined in such a way that the bottom (10) of the ring (2) has at least one hole (16) passing through it.

8. The method as claimed in the immediately preceding claim, in which the hole (16) is defined in such a way that, in the service position, the bottom of the ring does not even partially cover the mastication surface of the first fixation tooth.

9. The method as claimed in any one of the preceding claims, said method having, after step c), a step d) in which at least part of the outer surface of the bracket member is polished, without the inner surface of the bracket member being polished.

10. The method as claimed in any one of the immediately preceding claims, in which, in step b), the ring model is defined in such a way that the height of the lingual wall (12₁) and/or of the vestibular wall (12₂) is greater than the height of the first proximal wall and/or of the second proximal wall.

11. The method as claimed in the immediately preceding claim, in which, in step b), the ring model is defined in such a way that the lingual wall (12₁) and/or the vestibular wall (12₂) extend, from the bottom (10), beyond the line of greatest contour (Lₘₐₓ) of the fixation tooth.

12. The method as claimed in any one of the immediately preceding claims, in which, in step b), when the first proximal wall is intended to extend, in the service position, between the first fixation tooth (D_{f}) and an adjacent tooth (Dₐ) in contact with the first fixation tooth via a contact zone (Z_{c}), the ring model is defined in such a way that, in said service position, the first proximal wall does not reach said contact zone.

13. The method as claimed in any one of the preceding claims, in which the active member (21) is made of said shape-memory material or of said polymer material deformable under the effect of an activation stimulus.

14. The method as claimed in any one of the preceding claims, in which, after step c), glue or a sealing cement is applied to the inner surface (14ᵢ) of the ring.

15. The method as claimed in claim 14, claim 6 applying, in which the quantity of glue or sealing cement is adjusted in order to fill the region which, beyond the line of greatest contour, extends between the inner surface of the ring and the surface of the fixation tooth.
